# EUROPEAN PATENT APPLICATION

(11) **EP 2 158 938 A1**
(43) Date of publication of application: **03.03.2010**
(21) Application number: 09252077.4
(22) Date of filing: 27.08.2009
(51) Int. Cl.: A61N 1/365, A61B 5/00, A61B 19/00, A61N 1/05, A61N 1/362

(54) **Pacemaker with position sensing**

(30) Priority: 28.08.2008 US 200297
(71) Applicant: Biosense Webster, Inc., Diamond Bar, CA 91765 (US)
(72) Inventor: Govari, Assaf, Haifa 34400 (IL); Schwartz, Yitzhack, Haifa 34606 (IL)
(74) Representative: Small, Gary James

(57) **Abstract**

A pacemaker with position sensing capability permits built-in monitoring of hemodynamic changes. A miniature position sensor, such as a magnetic coil, is fixed to each implanted pacing lead. The pacemaker housing contains a generator unit, including a magnetic field transmitter. The magnetic field transmitted by the generator unit causes the position sensors to generate position signals, which are returned via the pacing leads to a control unit of the pacemaker. Based on these signals, the control unit senses relative positions of the location sensors, and hence the motion of the leads in the heart. Other location sensing techniques are also disclosed.

## Description

### BACKGROUND OF THE INVENTION

### Field of the Invention

This invention relates to intrabody tracking systems. More particularly, this invention relates a pacemaker with position sensing capability.

### Description of the Related Art

Conventional pacemaker systems include a cardiac stimulator and an elongated flexible lead that is connected proximally to a header structure on the cardiac stimulator. The lead is implanted distally at one or more sites within the heart requiring cardiac stimulation or sensing. At the time of implantation, the distal end of a pacemaker lead is inserted through an incision in the chest and manipulated to a site requiring electrical stimulation. Then, the distal end of the lead is anchored to the endocardium, using various devices, such as a screw tip, or tines that engage the myocardium. The proximal end of the lead is then connected to the header and the incision is closed.

When a heart is paced by such a system, its hemodynamic efficiency is generally reduced relative to the heart in normal sinus rhythm. One of the purposes of multi-chamber pacing is hemodynamic improvement by controlling the relative timing of the contraction of different chambers of the heart. In some cases, the treating physician programs the pacemaker to achieve optimal hemodynamic effect. Hemodynamics of the paced heart may change over time, however, due to various factors.

U.S. Patent No. 6757563 to Sweeney proposes a cardiac rhythm management system that provides ultrasound autocapture capability for determining whether a stimulation has evoked a desired response from the heart, and for adjusting an energy of the stimulation based on the observed response from the heart. An autocapture determination circuit determines whether motion of the heart chamber indicates a contraction in response to the stimulation, and adjusts the stimulation energy to provide only that energy which is needed to ensure reliable capture.

### SUMMARY OF THE INVENTION

According to disclosed embodiments of the invention, a pacemaker with position sensing capability permits built-in monitoring of hemodynamic changes. An arrangement of transducers and sensors are incorporated in the leads and the housing.

In one embodiment, a miniature position sensor, such as a magnetic coil, is fixed to each of the pacing leads, which are implanted in the heart. The pacemaker housing itself contains a generator unit, which can be a magnetic field transducer. The magnetic field transmitted by the generator unit causes the position sensors to generate position signals, which are returned via the pacing leads to a control unit of the pacemaker. Based on these signals, the control unit senses the positions of the location sensors, relative to the generator unit, and hence the motion of the leads in the heart.

Other sensing techniques can be used to obtain position information of the leads, for example impedance measurements between the lead and the generator unit.

The control unit compares the motion data to baseline data stored previously. Alternatively, the control unit may transmit the motion data to an external telemetry unit, which performs the comparison. If the pattern of motion has changed significantly relative to the baseline, it may be a sign that the cardiac hemodynamics have changed. In this case, the control unit or telemetry unit issues an alert to indicate that adjustment of the pacemaker or other therapy may be needed.

An embodiment of the invention provides a cardiac pacemaker apparatus, including a housing implantable in a living subject, and at least one stimulating lead extending from the housing. The lead has a distal segment adapted for engagement with the subject's heart, and a location detection unit for determining position coordinates of the distal segment of the lead, which is adapted for containment within the subject's body.

According to an aspect of the apparatus, the location detection unit includes a first element associated with the lead and a second element in the housing. The location detection unit is operative to determine a location of the first element relative to the second element.

According to one aspect of the apparatus, the location detection unit includes a location sensor in the distal segment, a generator unit adapted to generate field signals for reception thereof by the location sensor, and a processor operative to receive location signals generated by the location sensor responsively to the field signals.

According to another aspect of the apparatus, the location detection unit also includes a conversion unit for computing location coordinates of the distal segment relative to the generator unit responsively to the location signals.

According to a further aspect of the apparatus, the location detection unit also includes a telemetry unit for transmitting telemetry data derived from the location signals to a position processor that is disposed outside the subject.

According to an additional aspect of the apparatus, the location detection unit includes a magnetic field generator in the distal segment, a location sensor outside of the distal segment adapted to receive field signals for from the magnetic field generator, and a processor operative to receive location signals generated by the location sensor responsively to the field signals.

According to still another aspect of the apparatus, the magnetic field generator has no more than one generator coil.

According to an additional aspect of the apparatus, the location sensor includes no more than one receiving coil.

According to still another aspect of the apparatus, the location detection unit includes a conversion unit for computing location coordinates of the distal segment relative to the location sensor responsively to the location signals.

According to yet another aspect of the apparatus, the location detection unit also includes a telemetry unit for transmitting telemetry data derived from the location signals to a position processor that is disposed outside the subject.

According to yet another aspect of the apparatus, the location detection unit also includes a telemetry unit for transmitting telemetry data derived from the location signals to a position processor that is disposed outside the subject.

According to a further aspect of the apparatus, the location detection unit includes a signal electrode in the distal segment, driving circuitry to provide driving signals to the signal electrode via the lead, a plurality of conductive elements, and a processor linked to the conductive elements and operative to determine respective impedances between the signal electrode and the conductive elements responsively to the driving signals.

According to another aspect of the apparatus, the location detection unit also includes a conversion unit for computing location coordinates of the distal segment relative to the respective conductive elements responsively to the impedances.

According to one aspect of the apparatus, the location detection unit also includes a telemetry unit for transmitting telemetry data derived from the impedances to a position processor that is disposed outside the subject, the position processor is operative for computing location coordinates of the distal segment relative to the conductive elements responsively to the impedances.

Another embodiment of the invention provides a method for carrying out the functions of the above-described apparatus.

### BRIEF DESCRIPTION OF THE DRAWINGS

For a better understanding of the present invention, reference is made to the detailed description of the invention, by way of example, which is to be read in conjunction with the following drawings, wherein like elements are given like reference numerals, and wherein:

Fig. 1 is a pictorial diagram of a pacemaker system implanted in a living subject in accordance with a disclosed embodiment of the invention;

Fig. 2 is a flow chart of a method for evaluation of cardiac function using a pacemaker system in accordance with a disclosed embodiment of the invention;

Fig. 3 is a detailed schematic drawing of an embodiment of the pacemaker system illustrated in Fig. 1, which is constructed and operative in accordance with a disclosed embodiment of the invention;

Fig. 4 is a flow chart illustrating a method of determining position and orientation of an object, in accordance with a disclosed embodiment of the invention;

Fig. 5 is a schematic drawing showing an embodiment of a pacemaker system, which is constructed and operative in accordance with an alternate embodiment of the invention;

Fig. 6 is a detailed schematic drawing showing an embodiment of a pacemaker system, which is constructed and operative in accordance with an alternate embodiment of the invention;

Fig. 7 is a detailed schematic drawing showing an embodiment of a pacemaker system, which is constructed and operative in accordance with an alternate embodiment of the invention; and

Fig. 8 is a detailed schematic drawing showing an embodiment of a pacemaker system, which is constructed and operative in accordance with an alternate embodiment of the invention.

### DETAILED DESCRIPTION OF THE INVENTION

In the following description, numerous specific details are set forth in order to provide a thorough understanding of the present invention. It will be apparent to one skilled in the art, however, that the present invention may be practiced without these specific details. In other instances, well-known circuits, control logic, and the details of computer program instructions for conventional algorithms and processes have not been shown in detail in order not to obscure the present invention unnecessarily.

### Embodiment 1

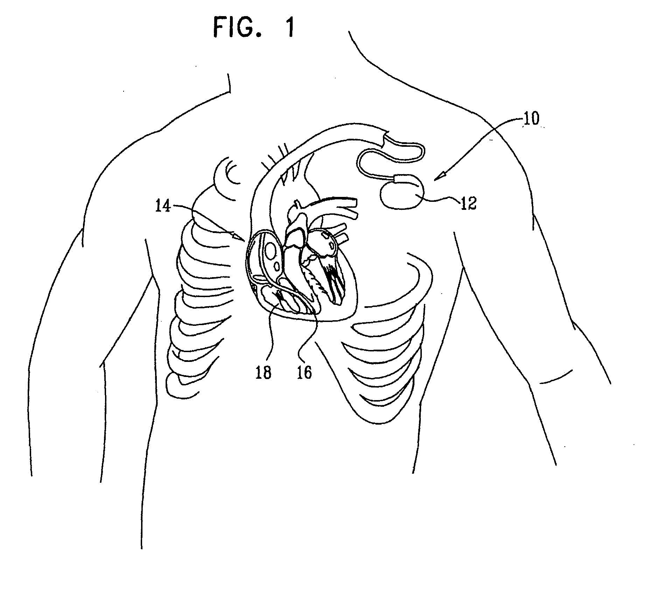

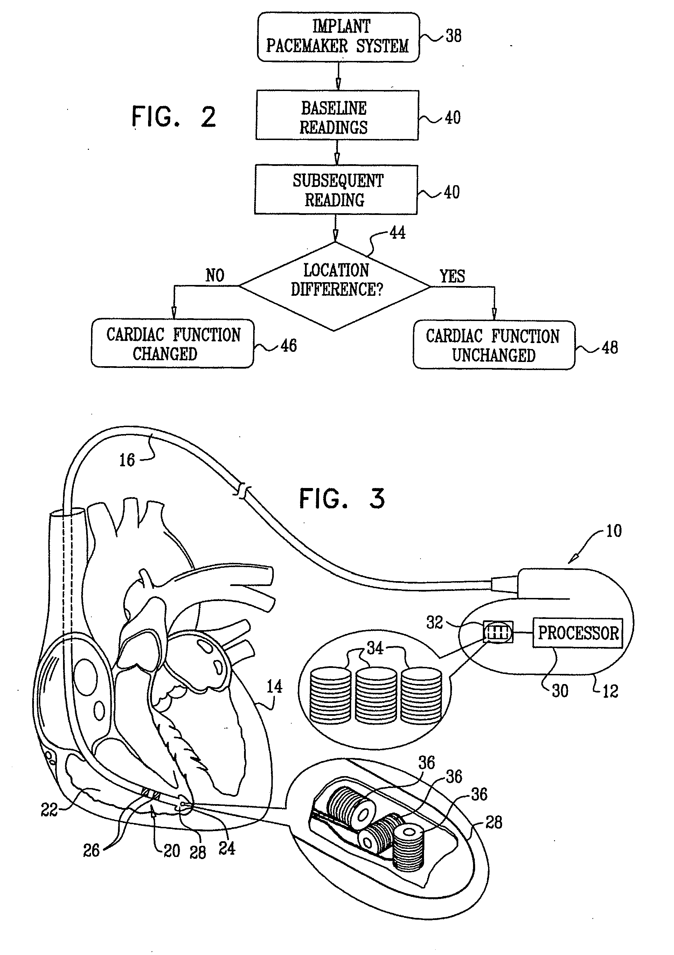

Turning now to the drawings, reference is initially made to Fig. 1, which is a pictorial diagram of a pacemaker system 10 implanted in a living subject in accordance with a disclosed embodiment of the invention. A pacemaker housing 12 has been implanted subcutaneously. The pacemaker housing 12 contains any suitable conventional circuitry (not shown) for stimulating a heart 14. A lead 16 extends from the pacemaker housing 12 into a right ventricular chamber 18 of the heart 14, where it is implanted. Although one lead is shown representatively in Fig. 2, there can be more than one lead, placed in different locations or different chambers within the heart 14.

A conventional cardiac stimulator lead normally consists of an elongated flexible tubular, electrically insulating sleeve, connected proximally to a connector that is adapted to couple to the header of a cardiac stimulator such as the pacemaker housing 12. Distally there is a tubular tip electrode. Additionally or alternatively, one or more ring electrodes may be secured to the sleeve at various positions along the length of the sleeve. The proximal end of the sleeve is connected to the connector by application of various biocompatible adhesives to various portions of the connector and the sleeve. The tip electrode ordinarily consists of a tubular structure that has a section whose diameter is increased, and which forms an annular shoulder against which the distal end of the lead sleeve is abutted. The exterior surface of the tubular structure is normally smooth as is the interior surface of the distal end of the lead sleeve. Many such leads known in the art are suitable for use as the lead 16, for example the leads disclosed in U.S. Patent No. 5,851,227, issued to Spehr, and U.S. Patent No. 6,167,314, issued to Fischer Sr., et al, whose disclosures are herein incorporated by reference.

Reference is now made to Fig. 3, which is a schematic drawing showing in greater detail an embodiment of the pacemaker system 10, which is constructed and operative in accordance with a disclosed embodiment of the invention. A distal end 20 of the lead 16 is disposed in the heart 14 near the apex of its right ventricular chamber 22. The distal end 20 carries a pacing tip electrode 24 and pacing ring electrodes 26. These electrodes are conventional. Disposed in the distal end 20 is a miniature location sensor 28. In one embodiment, signals generated in the location sensor 28 are conducted to a position processor 30, which is disposed in the pacemaker housing 12. Also contained within the pacemaker housing 12 is a generator unit 32, which generates a plurality of electromagnetic fields using magnetic field generators 34, which are typically realized as coils.

Construction and operation of field generators suitable for use as the field generators 34 are generally described in U.S. Patent No. 5,729,129, which is herein incorporated by reference. It will be understood that placement of the field generators 34 in the generator unit 32, as well as their size and shape, is necessarily different from the embodiment described in U.S. Patent No. 5,729,129. Furthermore, as the sensing volume covered by the pacemaker system 10 is considerably reduced from the exemplary applications shown, the power requirements of driving circuitry used to power the field generators 34 may be reduced accordingly. Power levels of about 10-30 mW are suitable. In typical modes of operation of the pacemaker system 10, the field generators 34 only need to be activated intermittently, which makes power storage for them in the pacemaker housing 12 practical. A typical lithium type battery is expected to last for more than a week in normal service, assuming a duty cycle of 10%.

In one embodiment, the location sensor 28 is an electromagnetic position sensor, which receives electromagnetic field signals from the generator unit 32. The electromagnetic fields are generated in order to define a frame of reference for tracking the position of the distal end 20 of the lead 16. Thus, based on sensed electromagnetic fields, the location sensor 28 transmits a location signal to the processor 30, and can provide at least five dimensions of position information (X, Y, Z, pitch and yaw) in the form of coordinate information. Alternatively, six dimensions of position and even orientation information (X, Y, Z, pitch, yaw and roll) may be provided. As noted above, the location signal is conducted through the lead 16.

As stated, sensors sensing fewer than six degrees of location information may be used. For example, a sensor, which senses five degrees of location information (three position coordinates, pitch and yaw) is described in U.S. Pat. No. 5,913,820, the disclosure of which is incorporated herein by reference. Alternatively, a plurality of location sensors, each providing less than six degrees of location information, may be used. For example, three or more location sensors, each providing three degrees of location information, may be used to define the location of all points on the lead 16.

Understanding of the instant invention will be facilitated by a brief description of a locating and mapping system, elements of which are incorporated in the generator unit 32, and used to track the position of the location sensor 28, from which the position of the tip of the lead 16, having a known offset from the location sensor 28 can be readily derived. A suitable location and mapping subsystem is disclosed in U.S. Patent Nos. 5,840,025, 5,391,199 and 6,690,963, which are herein incorporated by reference. The location sensor 28 is typically an alternating current (AC) magnetic field receiver that senses the magnetic fields generated by the generator unit 32. These transmitters generate AC magnetic fields to define a fixed frame of reference. A suitable sensor for use as the location sensor 28 is further described in the above-noted U.S. Patent No. 5,391,199. The position coordinates of the location sensor 28 are then ascertained by determining its position coordinates. The location sensor 28 may comprise one or more antennas, for example one or more coils 36.

The field generators 34 are driven by driving circuits (not shown) controlled by the processor 30. The signals received from the location sensor 28 are amplified and processed, together with a representation of the driving signals by the processor 30 to provide an indication of the position of the distal end 20. When driven, the field generators 34 generate a multiplicity of distinguishable AC magnetic fields that are sensed by the location sensor 28. The magnetic fields are distinguishable with regard to the frequency, phase, or both frequency and phase of the signals in the respective magnetic fields. Time multiplexing of the different magnetic fields is also possible.

The location sensor 28 may consist of a single coil, together with a single field generator 34. But more commonly it has two or more and even three sensor coils wound on either air cores or a core of material. The pacemaker housing 12 can accommodate two or three field generators 34. When a plurality of coils 36 are used, they preferably have mutually orthogonal axes, one of which is conveniently aligned with the longitudinal axis of the lead 16. The coils 36 are either interconnected, or can be closely spaced along the longitudinal axis of the lead 16 to reduce the diameter of the location sensor 28.

For most aspects of the present invention, the position of the distal end 20 relative to a reference frame is measured quantitatively. A reference frame is provided in the generator unit 32 This fixed frame of reference is provided by non-overlapping field generators 34 that generate at least two distinguishable AC magnetic fields for reception by the location sensor 28. Preferably, there should be at least two non-parallel coils 36 in the location sensor 28 to measure the magnetic field flux resulting from the distinguishable magnetic fields. To determine six position and coordinates (X, Y, Z directions and pitch, yaw and roll orientations), it is desirable that at least two coils 36 and three transmitters in the generator unit 32. Three coils would typically be used to improve the accuracy and reliability of the position measurement. In other applications, where fewer position coordinates are required, only a single coil may be necessary in the location sensor 28.

Specific features and functions of a single axis positioning system having only one coil are described in commonly assigned U.S. Patent 6,484,118, which is incorporated herein by reference. In one embodiment the coils 36 have an inner diameter of 0.5 mm and have 800 turns of 16 micrometer diameter to give an overall coil diameter of 1-1.2 mm. The effective capture area of each coil is typically about 400 mm². It will be understood that these dimensions may vary over a considerable range. In particular, the size of the coils 36 can be as small as 0.3 mm (with some loss of sensitivity) and can exceed 2 mm. The wire size of the coils 36 can range from 10-31 micrometers, and the number of turns may vary between 300 and 2600, depending on the maximum allowable size and the wire diameter. The effective capture area should be made as large as feasible, consistent with the overall size requirements. While the usual sensor coil shape is cylindrical, other shapes can also be used. For example, a barrel-shaped coil can have more turns than a cylindrical coil of the same diameter.

The processor 30 converts readings data read from the location sensor 28 to position data and optionally orientation data. As explained below, a succession of readings from the location sensor 28 in a session are used to construct motion data, which are stored. The processor 30 may compare the readings and motion data with corresponding data obtained previously and stored. Alternatively, the processor 30 may transmit raw data from the location sensor 28 to an external telemetry unit (not shown), which functions as a conversion unit and performs the conversions and comparisons. Alternatively, the location sensor 28 may convert raw data into position data and optionally orientation data, which is then transmitted to the external telemetry unit for further processing. If the pattern of motion has changed significantly relative to the baseline, it may be a sign that the cardiac hemodynamics have changed. In this case, the processor 30 or telemetry unit may issue an alert to indicate that adjustment of the pacemaker or other therapy may be needed.

### Operation

Reference is now made to Fig. 2, which is a flow chart of a method for evaluation of cardiac function using a pacemaker system in accordance with a disclosed embodiment of the invention. At initial step 38 a pacemaker system, e.g., the pacemaker system 10 is implanted in a living subject as shown generally in Fig. 1.

Next, at step 40 the field generators 34 are actuated, and baseline readings are taken from the location sensor 28 and converted to position data and optionally orientation data. Details of step 40 are given below.

Step 42 is performed subsequent to the performance of step 40. The field generators 34 are again actuated, a new set of readings are taken from the location sensor 28 and converted in the same manner as in step 40. Step 42 might be performed as part of a periodic patient monitoring protocol. Alternatively, step 42 could be performed following some diagnostic or therapeutic manipulation, e.g., medication administration, change in medication, or an exercise stress test. Indeed, in embodiments in which the processor 30 transmits data to an external unit, step 42 might be performed at a site remote from the site of step 40, e.g., the patient's home.

Control now proceeds to decision step 44, where it is determined if position data and optional orientation data of step 40 differ meaningfully from corresponding data obtained in step 42. If the determination at decision step 44 is affirmative, then control proceeds to final step 46. It is concluded that the patient's cardiac function has changed.

If the determination at decision step 44 is negative, then control proceeds to final step 48. It is concluded that the patient's cardiac function has not changed.

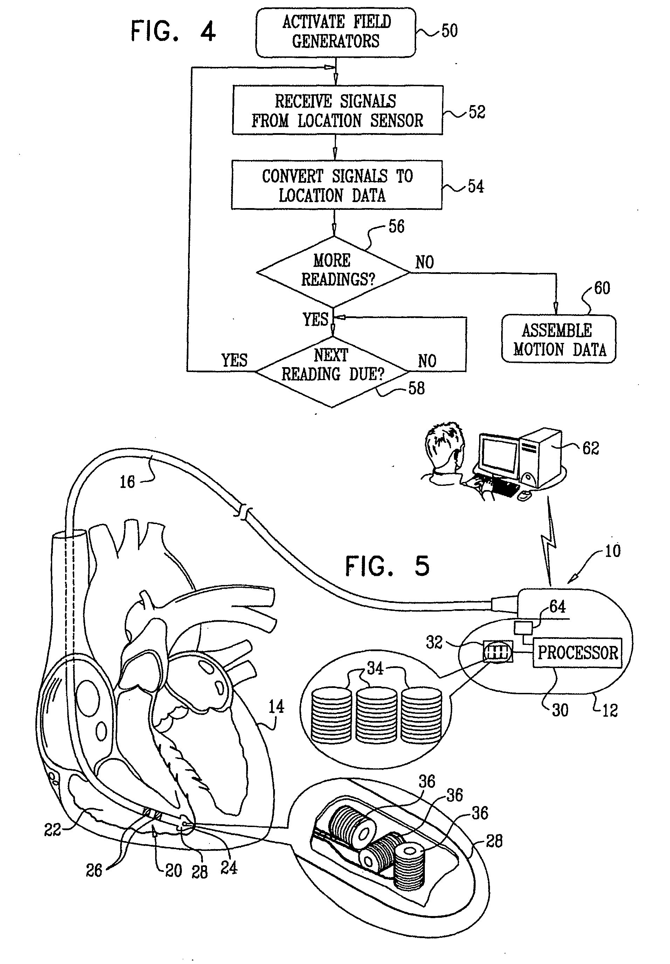

Reference is now made to Fig. 4, which is a flow chart illustrating a method of determining a position and/or orientation of an object relative to a reference location, in accordance with a disclosed embodiment of the invention. The method described with reference to Fig. 4 is performed in steps 40, 42 (Fig. 2). The process steps are shown in a particular linear sequence in Fig. 4 for clarity of presentation. However, it will be evident that many of them can be performed in parallel, asynchronously, or in different orders.

At initial step 50 field generators, e.g., field generators 34 (Fig. 3) are actuated.

Next, at step 52, at a predetermined point of the cardiac cycle, signals developed in the location sensor 28 (Fig. 3) are gated into the processor 30. Alternatively, the processor 30 may receive the signals continuously and accept them for further processing only at predetermined intervals, or predetermined times in the cardiac cycle.

Next, at step 54, the signals received in initial step 50 are converted into position data and optionally orientation data, as described above. The converted data is stored. Additionally or alternatively, raw data embedded in the received signals may be stored for subsequent conversion.

Control now proceeds to decision step 56, where it is determined if all predetermined intervals, or alternatively all predetermined points of the cardiac cycle have been evaluated. If the determination at decision step 56 is negative, then control proceeds to delay step 58, wherein the next predetermined point in time or in the cardiac cycle is awaited. Control then returns to step 52.

If the determination at decision step 56 is affirmative, then control proceeds to final step 60. Here the periodic data obtained in steps 52, 54 may be assembled into a motion image or graph, suitable for comparison with another such image or graph. The field generators may now be deactivated to conserve power. The process terminates.

### Embodiment 2

Reference is now made to Fig. 5, which is a schematic drawing showing an embodiment of the pacemaker system 10, which is constructed and operative in accordance with an alternate embodiment of the invention. In this embodiment, an external position processor 62 is provided, which receives data from the processor 30 by telemetry from a telemetry unit 64 installed in the pacemaker housing 12. Suitable units for the telemetry unit 64 are known, for example, from U.S. Patent No. 6,239,724 to Doron et al, which is herein incorporated by reference. The position processor 62 includes a suitable receiver (not shown) for signals generated by the telemetry unit 64. The position processor 62 may receive raw data read from the location sensor 28 at suitable intervals under control of the processor 30. Alternatively, the processor 30 may transmit position data and optionally orientation coordinates relating to the location sensor 28, in which case the position processor 62 may refine the received data and prepare tabular or graphical presentations indicating motion of the location sensor 28 and hence the tip of the lead 16.

### Embodiment 3

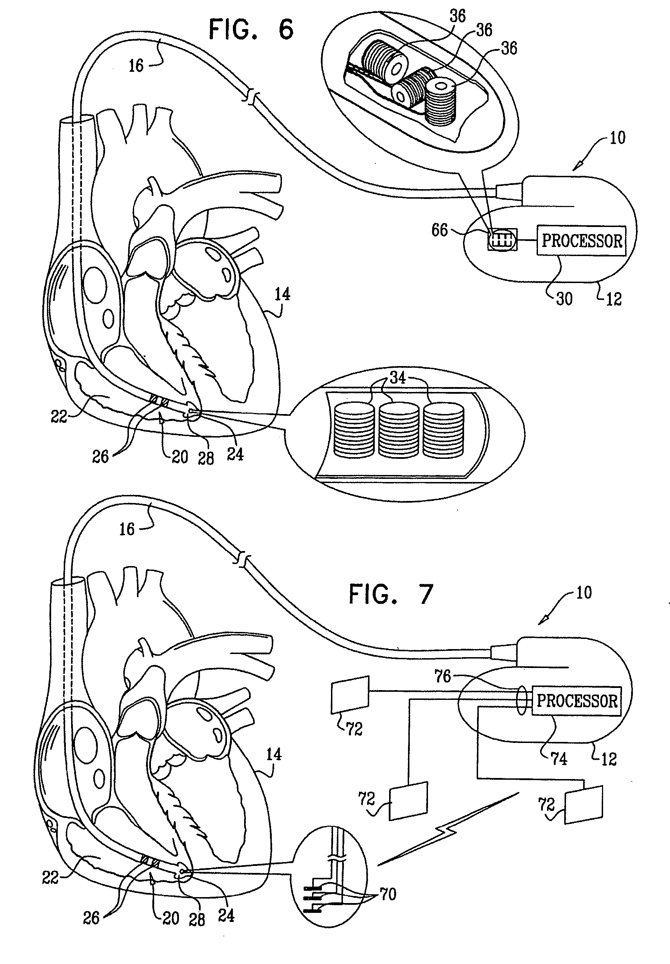

Reference is now made to Fig. 6, which is a schematic drawing showing an embodiment of the pacemaker system 10, which is constructed and operative in accordance with an alternate embodiment of the invention. In this embodiment, a receiver unit 66 now contains the coils 36, and acts as a receiver for magnetic fields, which are generated in a miniature transmitter 68. The transmitter 68 contains the field generators 34, and is powered via the lead 16. Signals received in the receiver unit 66 are processed in the processor 30 in any of the modes described above with respect to Embodiment 1 or Embodiment 2.

### Embodiment 4

Reference is now made to Fig. 7, which is a schematic drawing showing an embodiment of the pacemaker system 10, which is constructed and operative in accordance with an alternate embodiment of the invention. In this embodiment the location sensor 28 (Fig. 1) is omitted, and replaced by one or more signal electrodes 70. Interaction is shown between the electrodes 70 and reference conductive elements 72. The conductive elements 72 are implanted within the body of the subject, and linked to a control processor 74 by a cable 76. The processor 74 operates similarly to the processor 30 (Fig. 3), but now includes driving circuitry to provide driving signals to the electrodes 70 via the lead 16. Each of the electrodes 70 communicates with all of the conductive elements 72. The processor 30 drives a current between each of the electrodes 70 and all the conductive elements 72, and uses the current to measure the impedances between each of the electrodes 70 and the conductive elements 72. Based on the measured impedances, the processor 30 determines the position of the distal end 20 relative to the conductive elements 72. Alternatively, greater or smaller numbers of electrodes 70 may be used. For example, processor 30 may be set to multiplex the currents between one of the electrodes 70 multiple conductive elements 72. As another example, more than three conductive elements 72 may be used for enhanced accuracy.

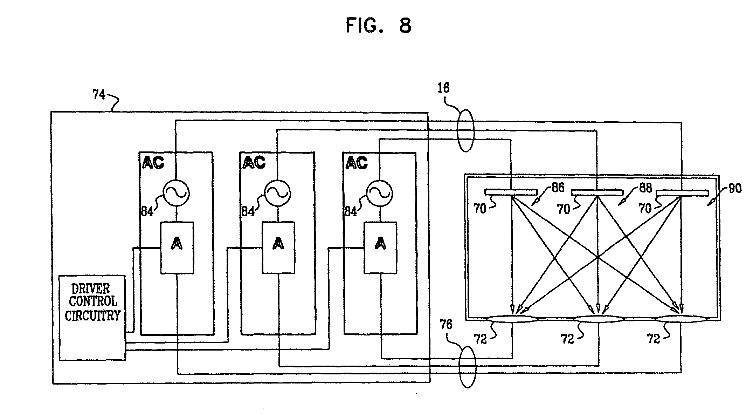

Reference is now made to Fig. 8, which is a block diagram showing elements of the pacemaker system 10 (Fig. 7) in accordance with an alternate embodiment of the invention. The processor 74 comprises control circuitry 78 for driving currents and for measuring impedance. The control circuitry 78 controls and monitors each of three circuits 80, 82, 84 drives a current through a closed loop consisting of one of the electrodes 70 and the conductive elements 72. Specifically, the three circuits 80, 82, 84 drive respective currents through different body tissues 86, 88, 90. Each of the currents generated by the driver circuits may be distinguished by setting the circuits 80, 82, 84 to operate at different frequencies.

Each of the circuits 80, 82, 84 measures the electrical impedance in its respective loop through the body tissues 86, 88, 90. These impedance readings are processed by the processor 74, which uses the readings to calculate the position coordinates of the distal end 20 relative to the conductive elements 72. Based on these position coordinates, the processor 74 or an external processing unit then generates motion information as described above. Further details of methods for using impedance measurements to obtain position coordinates are disclosed in U.S. Patent Nos. 5,697,377 and 5,983,126 to Wittkampf, and in commonly assigned, copending Application No. 11/213,040 filed on August 26, 2005, which are herein incorporated by reference.

It will be appreciated by persons skilled in the art that the present invention is not limited to what has been particularly shown and described hereinabove. Rather, the scope of the present invention includes both combinations and sub-combinations of the various features described hereinabove, as well as variations and modifications thereof that are not in the prior art, which would occur to persons skilled in the art upon reading the foregoing description.

## Claims

1. A cardiac pacemaker apparatus, comprising:
a housing implantable in a living subject;
at least one stimulating lead extending from said housing and having a distal segment adapted for engagement with a heart of said subject; and
a location detection unit adapted for containment within said subject for determining position coordinates of said distal segment of said lead.

2. The apparatus according to claim 1, wherein said location detection unit comprises a first element associated with said lead and a second element in said housing, and said location detection unit is operative to determine a location of one of said first element and said second element with respect to another of said first element and said second element.

3. The apparatus according to claim 1, wherein said location detection unit comprises:
a location sensor in said distal segment;
a generator unit adapted to generate field signals for reception thereof by said location sensor; and
a processor operative to receive location signals generated by said location sensor responsively to said field signals.

4. The apparatus according to claim 1, wherein said location detection unit comprises:
a magnetic field generator in said distal segment;
a location sensor outside of said distal segment adapted to receive field signals for from said magnetic field generator; and
a processor operative to receive location signals generated by said location sensor responsively to said field signals.

5. The apparatus according to claim 4, wherein said magnetic field generator comprises no more than one generator coil.

6. The apparatus according to claim 4, wherein said location sensor comprises no more than one receiving coil.

7. The apparatus according to claim 3 or claim 4, wherein said location detection unit further comprises a conversion unit for computing location coordinates of said distal segment relative to said generator unit/location sensor responsively to said location signals.

8. The apparatus according to claim 3 or claim 4, wherein said location detection unit further comprises a telemetry unit for transmitting telemetry data derived from said location signals to a position processor that is disposed outside said subject.

9. The apparatus according to claim 1, wherein said location detection unit comprises:
a signal electrode in said distal segment;
driving circuitry to provide driving signals to said signal electrode via said lead;
a plurality of conductive elements; and
a processor linked to said conductive elements and operative to determine respective impedances between said signal electrode and said conductive elements responsively to said driving signals.

10. The apparatus according to claim 9, wherein said location detection unit further comprises a conversion unit for computing location coordinates of said distal segment relative to said respective conductive elements responsively to said impedances.

11. The apparatus according to claim 9, wherein said location detection unit further comprises a telemetry unit for transmitting telemetry data derived from said impedances to a position processor that is disposed outside said subject, said position processor being operative for computing location coordinates of said distal segment relative to said conductive elements responsively to said impedances.

12. A method of assessing cardiac function, comprising the steps of:
providing a pacemaker apparatus comprising a housing and at least one stimulating lead extending from said housing, said lead having a distal segment adapted for engagement with a heart of a living subject;
implanting said housing in a living subject and engaging said distal segment in a heart of said subject;
disposing a location detection unit in said subject ; and
while said lead is engaged in said heart determining position coordinates of said distal segment of said lead using said location detection unit.

13. The method according to claim 12, wherein a portion of said location detection unit is disposed in said lead.

14. The method according to claim 12, further comprising the steps of:
iteratively performing said step of determining position coordinates to obtained a series of position coordinates; and
determining motion information of said distal segment of said lead from said series of position coordinates.

15. The method according to claim 12, wherein said location detection unit comprises a magnetic field generator and a location sensor responsive to signals produced by said magnetic field generator.

16. The method according to claim 12, wherein said location detection unit comprises an electrode, a plurality of conducting elements and a processor for measuring respective impedances between said electrode and said conducting elements.

17. The method according to claim 12, wherein said location detection unit comprises a first element associated with said lead and a second element in said housing, and said location detection unit is operative to determine a location of one of said first element and said second element with respect to another of said first element and said second element.
